# EUROPÄISCHE PATENTSCHRIFT

(11) **EP 1 568 336 B1**
(45) Veröffentlichungstag und Bekanntmachung des Hinweises auf die Patenterteilung: **14.11.2012**
(21) Anmeldenummer: 05001252.5
(22) Anmeldetag: 21.01.2005
(51) Int. Cl.: A61F 2/38

(54) **Gelenkersatz-Tibiaplateau**
Joint replacement for a tibial plate
Remplacement d'articulation du plateau tibial

(30) Priorität: 26.02.2004 DE 202004003133 U
(43) Veröffentlichungstag der Anmeldung: 31.08.2005
(73) Patentinhaber: LIMACORPORATE S.p.A., 33038 San Daniele del Friuli (UD) (IT)
(72) Erfinder: Nägerl, Hans, Prof. Dr., 37130 Gleichen (DE)
(74) Vertreter: Petraz, Gilberto Luigi

(56) Entgegenhaltungen:
- WO-A-94/09724
- DE-A1- 4 006 714
- GB-A- 2 120 943
- US-A- 4 808 185
- US-A- 5 137 536

## Beschreibung

Die Erfindung betrifft die Gestaltung des Tibiatableaus bei einem Gelenkersatz, insbesondere einer Endoprothese für das menschliche Kniegelenk.

Die Schriften DE 42 02 717 C1, DE 195 21 597 A1 und DE 196 46 891 A1 betreffen ein künstliches Gelenk, insbesondere eine Endoprothese zum Ersatz natürlicher Gelenke. Die Endoprothese besteht aus mindestens zwei künstlichen Gelenkteilen mit gekrümmten Artikulationsflächen, wobei auf jeder der Artikulationsflächen eine gekrümmte Kontaktlinie, speziell eine kreisbogenförmige Kontaktlinie ausgebildet ist. Die Artikulationsflächen sind derart als Paar zueinander angeordnet, dass die Kontaktlinien aufeinander abrollen können. Bei der Bewegung der Gelenkteile zueinander kann es zu einer zu weiten Abroll- und damit Schwenkbewegung der Gelenkteile zueinander und/oder zu einem Seitenversatz kommen, der zu Fehlfunktionen des Gelenks führen kann.

Der Erfindung liegt deshalb die Aufgabe zugrunde, das Implantat so zu gestalten, dass die Schwenkbewegung begrenzt bzw. der Seitenversatz verhindert wird, so dass eine indikationserweiternde, praktikable Sicherheitsbarriere vorliegt. Bei der konstruktiven Lösung soll zusätzlich zur Bewegungsbegrenzung der dimeren Gelenkkette eine mechanisch Begrenzung der funktionellen Bewegung erfolgen, wobei jedoch die ursprünglichen Funktionsflächen unveränder belassen bleiben.

Die Lösung der Aufgabe erfolgt entsprechend den Merkmalen des Anspruchs 1. Die Unteran-spüche geben zweckmäßige Ausgestaltungen der Lösung nach Anspruch 1 wieder Ein Gelenzersatz-Tibeaplateau gemäβ dem Oberbegriff des Anspruchs 1 wird in der US 5 137 536 offenbart. Die Erfindung soll an dem Ausführungsbeispiel einer Endoprothese für ein menschliches Kniegelenk anhand der Zeichnungen erläutert werden. In den Figuren der Zeichnungen stellen dar:

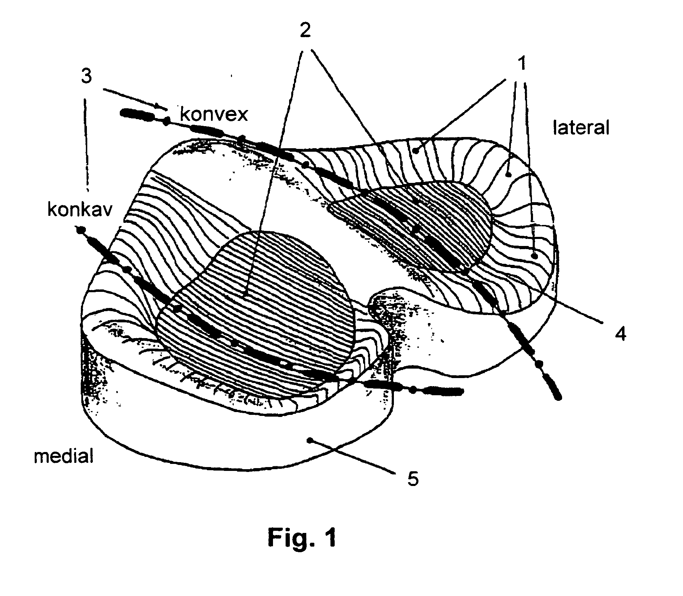

- Fig. 1:: Ansicht auf das Tibiaplateau eines Kniegelenkersatzes;
- Fig. 2:: seitliche Schnittdarstellung durch den medialen und lateralen Teil des Tibiaplateaus;
- Fig. 3:: seitliche Schnittdarstellung durch Gelenkkopf und Pfanne zur Darstellung der Bewegungsbegrenzung.

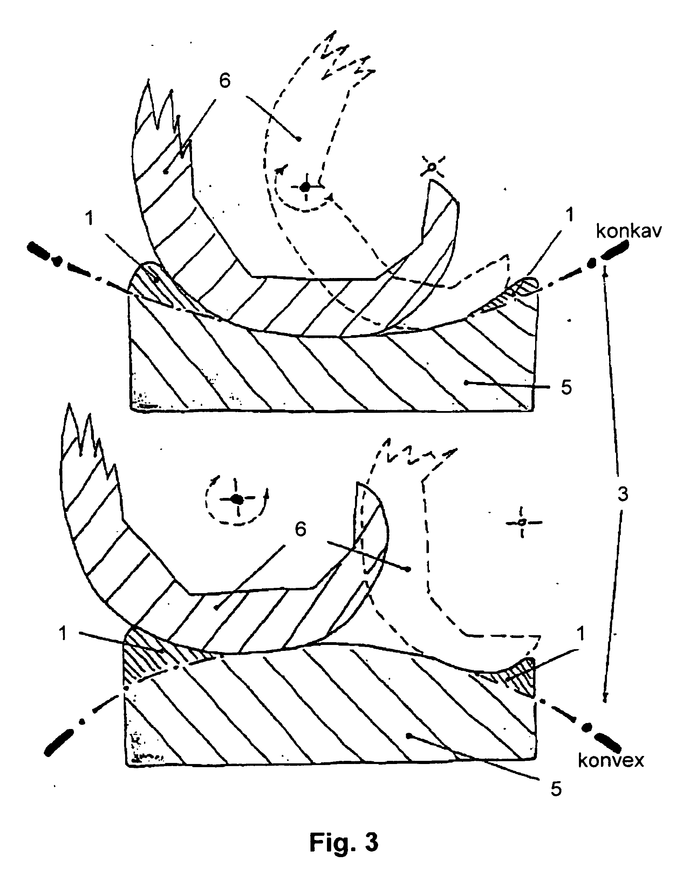

Das Tibiaplateau 5 der Endoprothese für das menschliche Kniegelenk schließt zwei Funktonsflächen 2 ein, von denen eine, entsprechend der Kennzeichnung 3, eine konvexe und die andere eine konkave Krümmung besitzt. An den Randbereich 4 der Funktionsflächen 2 schließt sich erfindungsgemäß eine allmählich ansteigende Wulst 1 an. Diese Wulst 1 bildet eine mechanische Begrenzung für den zweiten, Gelenkteil, im vorliegenden Fall den Femurgelenkersatz 6 (Fig. 3), dadurch, dass dieser bei Gelenkbewegung auf der Funktionsfläche 2 abrollt und durch Anschlag an der Wulst 1 an werterem Abrollen oder an Seitenversatz gehindert wird.

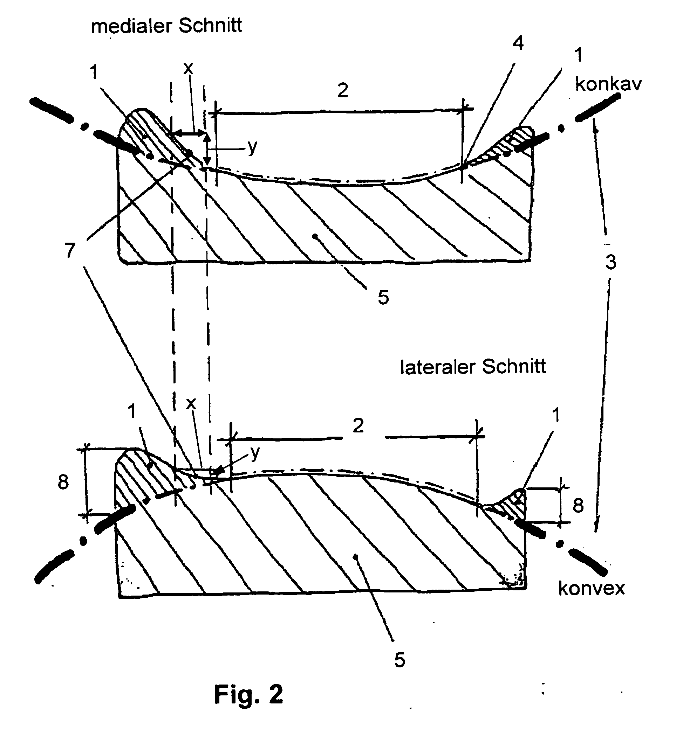

Die Schnittdarstellungen der Fig. 2 und 3 zeigen sowohl den medialen Schnitt mit der konkaven Krümmung als auch den lateralen Schnitt mit der konvexen Krümmung der Funktionsflächen 2. Die Wulst 1 kann, von der Funktionsfläche 2 aus gesehen, in allen Richtungen oder nur in bestimmten Richtungen, also nicht die Funktionsfläche 2 vollständig umschließend, ausgebildet sein. Der Anstieg 7 der Wulst 1 kann nach allen Richtungen gleich oder in unterschiedlichen Richtungen unterschiedlich sein. Der Anstieg 7 der Wulst 1 kann linear (y/x) oder mit konkaver Krümmung (dy/dx), nach allen Richtungen gleich oder in unterschiedlichen Richtungen unterschiedlich, erfolgen. Die Höhe 8 der Wulst 1 kann nach allen Richtungen gleich oder in unterschiedlichen Richtungen unterschiedlich sein.

Der praktische Einsatz der erfindungsgemäßen Wulst 1 am Tibiaplateau der Endoprothese hat gezeigt, dass erst mit dieser Maßnahme das künstliche Gelenk, wie es in den genannten Schriften zum Stand der Technik beschrieben ist, voll funktionsfähig gemacht werden konnte. Die Anwendung der erfindungsgemäßen Maßnahme ist nicht auf das Beispiel des künstlichen Kniegelenks beschränkt.

## Patentansprüche

1. Gelenkersatz-Tibiaplateau, insbesondere bei einer Endoprothese für das menschliche Kniegelenk, wobei die künstlichen Gelenkteile, Kopf und Pfanne, konvex bzw. konkav gekrümmte Artikulationsflächen besitzen, wobei auf jeder der Artikulationsflächen eine gekrümmte Kontaktlinie, insbesondere eine kreisbogenförmige Kontaktlinie, ausgebildet ist, und wobei die Artikulationsflächen derart als Paar zueinander angeordnet sind, dass die gebildeten Funktionsflächen aufeinander abrollen können, wobei auf dem sich an die Funktionsflächen (2) anschließenden Bereich (4) eine allmählich ansteigende Wulst (1) ausgebildet ist und die Anstiegslinie (7) der Wulst (1) mit einer Krümmung verläuft, **dadurch gekennzeichnet, dass** das Tibiaplateau (5) zwei Funktionsflächen (2) einschließt, von denen die eine eine konvexe und die andere eine konkave Krümmung besitzt und die Anstiegslinie (7) der Wulst (1) mit konkaver Krümmung verläuft, wobei die Wulst (1) in unterschiedlichen Richtungen einen unterschiedlichen Anstieg (7) besitzt und die Höhe (8) der Wulst (1) in unterschiedlichen Richtungen unterschiedlich ist.

2. Gelenkersatz-Tibiaplateau nach Anspruch 1, **dadurch gekennzeichnet, dass** die Wulst (1) von der Funktionsfläche (2) aus gesehen in allen Richtungen ausgebildet ist.

3. Gelenkersatz-Tibiaplateau nach Anspruch 1, **dadurch gekennzeichnet, dass** die Wulst (1) nicht in allen Richtungen ausgebildet ist.

4. Gelenkersatz-Tibiaplateau nach Anspruch 1 oder 2, **dadurch gekennzeichnet, dass** die Wulst (1) in allen Richtungen gleichen Anstieg (7) besitz.

5. Gelenkersatz-Tibiaplateau nach einem der vorangehenden Ansprüche 1 bis 3, **dadurch gekennzeichnet, dass** die Wulst (1) in unterschiedlichen Richtungen unterschiedlichen Anstieg (7) besitz.

6. Gelenkersatz-Tibiaplateau nach einem der vorangehenden Ansprüche, **dadurch gekennzeichnet, dass** die Höhe (8) der Wulst (1) in allen Richtungen gleich ist.

7. Gelenkersatz-Tibiaplateau nach einem der Ansprüche 1 bis 5, **dadurch gekennzeichnet, dass** die Höhe (8) der Wulst (1) in unterschiedlichen Richtungen unterschiedlich ist.

8. Gelenkersatz-Tibiaplateau nach einem der vorangehenden Ansprüche, **dadurch gekennzeichnet, dass** die Anstiegslinie (7) der Wulst (1) linear verläuft.

## Claims

1. Tibia plateau for a joint replacement, in particular for an endoprosthesis for the human knee joint, wherein the artificial joint parts, head and socket, have convex or concave curved articulation surfaces, wherein on each of the articulation surfaces a curved contact line, in particular a circular arch-shaped contact line, is formed, and wherein said articulation surfaces are arranged in pairs to each other, so that the formed functional surfaces can roll against one another, wherein a gently sloping bulge (1) is formed adjacent the area (4) of the functional surfaces (2) and the slope line (7) of the bulge (1) develops with a curvature, **characterized in that** the tibia plateau (5) comprises two functional surfaces (2), of which one has a convex curvature and the other has a concave curvature and the slope line (7) of the bulge (1) develops with a concave curvature, wherein the bulge (1) has a different slope (7) in different directions and the height (8) of the bulge (1) is different in different directions.

2. Tibia plateau for a joint replacement according to claim 1, **characterized in that** the bulge (1) is made in all directions as viewed from the functional surfaces (2).

3. Tibia plateau for a joint replacement according to claim 1, **characterized in that** the bulge (1) is not made in all directions.

4. Tibia plateau for a joint replacement according to claim 1 or 2, **characterized in that** the bulge (1) has the same slope (7) in all directions.

5. Tibia plateau for a joint replacement according to any claims from 1 to 3, **characterized in that** the bulge (1) has a different slope (7) in different directions.

6. Tibia plateau for a joint replacement according to any claims hereinbefore, **characterised in that** the height (8) of the bulge (1) is the same in all directions.

7. Tibia plateau for a joint replacement according to any claims from 1 to 5, **characterized in that** the height (8) of the bulge (1) is different in different directions.

8. Tibia plateau for a joint replacement according to any claims hereinbefore, **characterized in that** the slope line (7) of the bulge (1) develops linearly.

## Revendications

1. Plateau tibial d'une prothèse articulaire, en particulier dans le cas d'une endoprothèse pour l'articulation du genou humain, les parties de l'articulation artificielle, la tête et l'emboîture, possédant des surfaces articulaires à courbure convexe ou concave, une ligne de contact incurvée, en particulier une ligne de contact en forme d'arc de cercle, étant réalisée sur chacune des surfaces articulaires, les surfaces articulaires étant disposées l'une par rapport à l'autre sous la forme de paires de telle sorte que les surfaces fonctionnelles formées puissent se dérouler l'une sur l'autre, un renflement (1), à pente progressive, étant configuré sur la zone (4) qui suit les surfaces articulaires (2), et la pente (7) du renflement (1) présentant une courbure, **caractérisée en ce que** le plateau tibial (5) comprend deux surfaces fonctionnelles (2), dont l'une présente une courbure convexe et l'autre une courbure concave, et la pente (7) du renflement (1) présente une courbure concave, le renflement (1) présentant une pente différente (7) dans les différentes directions, et la hauteur (8) du renflement (1) étant différente dans les différentes directions.

2. Prothèse articulaire du plateau tibial selon la revendication 1, **caractérisée en ce que** le renflement (1), quand on regarde à partir de la surface fonctionnelle (2), est configuré dans toutes les directions.

3. Prothèse articulaire du plateau tibial selon la revendication 1, **caractérisée en ce que** le renflement (1) n'est pas configuré dans toutes les directions.

4. Prothèse articulaire du plateau tibial selon la revendication 1 ou 2, **caractérisée en ce que** le renflement (1) présente la même pente (7) dans toutes les directions.

5. Prothèse articulaire du plateau tibial selon l'une des revendications précédentes 1 à 3, **caractérisée en ce que** le renflement (1) présente une pente (7) différente dans les différentes directions.

6. Prothèse articulaire du plateau tibial selon l'une des revendications précédentes, **caractérisée en ce que** la hauteur (8) du renflement (1) est identique dans toutes les directions.

7. Prothèse articulaire du plateau tibial selon l'une des revendications 1 à 5, **caractérisée en ce que** la hauteur (8) du renflement (1) est différente dans les différentes directions.

8. Prothèse articulaire du plateau tibial selon l'une des revendications précédentes, **caractérisée en ce que** la ligne de pente (7) du renflement (1) est linéaire.
